# EUROPÄISCHE PATENTANMELDUNG

(11) **EP 1 013 637 A2**
(43) Veröffentlichungstag der Anmeldung: **28.06.2000**
(21) Anmeldenummer: 99124075.5
(22) Anmeldetag: 10.12.1999
(51) Int. Cl.: C07C 213/08, C07C 209/62, C07C 209/70, C07D 209/48, C07D 405/06, C07D 307/82, C07D 317/46, C07D 319/20, C07D 207/40, C07C 211/27

(54) **Verfahren zur Herstellung von Fluor enthaltenden Phenethylaminen sowie Fluor enthaltende Beta-Iminovinyl- und Beta-Iminoethylbenzole**

(30) Priorität: 23.12.1998 DE 19859684
(71) Anmelder: Bayer Aktiengesellschaft, 51368 Leverkusen (DE)
(72) Erfinder: Müller, Peter, Dr., 51467 Bergisch Gladbach (DE); Marhold, Albrecht, Dr., 51373 Leverkusen (DE)

(57) **Zusammenfassung**

Die vorliegende Erfindung betrifft ein Verfahren zur Herstellung von Fluor enthaltenden Phenethylaminen, das dadurch gekennzeichnet ist, daß man in einer ersten Stufe ein substituiertes Brombenzol mit einem N-Vinylimid in Gegenwart eines Palladiumkatalysators umsetzt, in einer zweiten Stufe das erhaltene substituierte β-Iminovinylbenzol katalytisch hydriert und in einer dritten Stufe das in der zweiten Stufe erhaltene substituierte β-Iminoethylbenzol spaltet. Mit diesem Verfahren werden auch neue β-Iminovinyl- und β-Iminoethylbenzole zugänglich.

## Beschreibung

Die vorliegende Erfindung betrifft ein neues Verfahren zur Herstellung von Fluor enthaltenden Phenethylaminen und dabei auftretende neue chemische Verbindungen.

4-Fluorphenethylamin und andere fluorhaltige substituierte Phenethylamine sind interessante Zwischenprodukte z.B. für die Herstellung von Agrochemikalien. Aus J. Am. Chem. Soc. 63, 602 (1941) ist bekannt, daß man 4-Fluorphenethylamine in einem vielstufigen Prozeß herstellen kann. Dabei wird ausgehend von p-Fluorphenylmagnesiumbromid durch Addition an Ethylenoxid mit nachfolgender Hydrolyse p-Fluorphenethylalkohol erhalten, der mit Phosphortribromid in p-Fluorphenylethylbromid umgewandelt wird, das man mit Ammoniak zum Zielprodukt verseift. Nachteilig für eine Anwendung dieses Verfahrens, vor allem im größeren Maßstab, sind neben der Vielstufigkeit der technische Aufwand zur Durchführung einer Grignard-Reaktion, inklusive der notwendigen sicherheitstechnischen Maßnahmen, sowie der Einsatz von Phosphortribromid, das kostenintensiv ist und für seine Handhabung ebenfalls großen sicherheitstechnischen Aufwand erfordert. Alternativ kann nach J. Org. Chem. 23, 1979 (1958) auch von p-Fluorbenzylchlorid ausgehen, dieses mit Natriumcyanid zum p-Fluorphenylessigsäurenitril umsetzen und dieses dann mit Natriumalanat reduzieren. Wenn man diese Route in den technischen Maßstab umsetzen würde, müßte man für den Einsatz des hochgiftigen Natriumcyanids besondere sicherheitstechnische Vorkehrungen treffen. Der Einsatz von Natriumalanat, das bekanntermaßen mit Halogenkohlenwasserstoffen explosionsartig reagiert (siehe Römpp Lexikon Chemie Version 1.3 auf CD-ROM (1997)) macht dieses Verfahren für eine technische Anwendung völlig unbrauchbar.

N-[2-(Fluorphenyl)-ethenyl]-phthalimid ist aus J. Org. Chem. 58, 3299 (1993) bekannt. Es wird dort als aus einer Jodverbindung zugänglich beschrieben. Dieses Verfahren ist unwirtschaftlich. Es konnte nicht erwartet werden, daß dieses und andere Fluor-enthaltende β-Iminovinylbenzole auf einfache Weise zugänglich sein könnten.

Es wurde nun ein Verfahren zur Herstellung von Fluor enthaltenden Phenethylaminen der Formel (I) gefunden 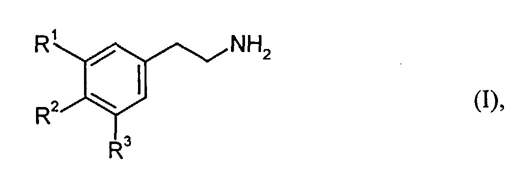 in der von den Resten
- R¹ und R²: einer für Fluor, Trifluormethyl, Trifluormethoxy, Tetrafluorethoxy oder Pentafluorethoxy und der andere für Wasserstoff, C₁ -C₆-Alkyl, C₁ -C₆-Alkoxy, Fluor, Trifluormethyl, Trifluormethoxy, Tetrafluorethoxy oder Pentafluorethoxy steht oder
- R¹ und R²: gemeinsam für -O-CF₂-O-, -O-CF₂-CF₂- oder -O-CF₂-CF₂-O- stehen und
- R³: für Wasserstoff, C₁-C₆-Alkyl oder C₁-C₆-Alkoxy steht,
das dadurch gekennzeichnet ist, daß man in einer ersten Stufe ein substituiertes Brombenzol der Formel 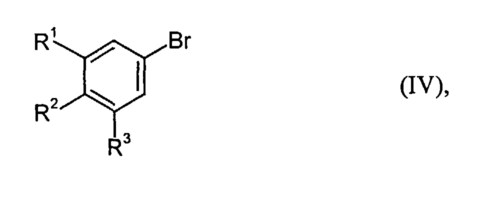 in der
- R¹, R² und R³: die bei Formel (I) angegebene Bedeutung haben,
mit einem N-Vinylimid der Formel 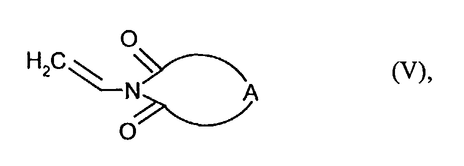 in der
- A: für -CH₂-CH₂-, -CH₂-CH₂-CH₂-, -CH₂-C(CH₃)₂-CH₂-, -CH₂-O-CH₂-, -CH=CH- oder o-Phenylen steht,
in Gegenwart eines Palladium-Katalysators umsetzt, in einer zweiten Stufe das erhaltene β-Iminovinylbenzol der Formel 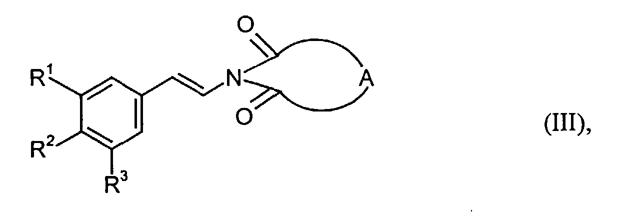 in der
die Reste R¹, R² und R³ die bei Formel (I) und der Rest A die bei Formel (V) angegebene Bedeutung haben,
katalytisch hydriert und in einer dritten Stufe das in der zweiten Stufe erhaltene substituierte β-Iminoethylbenzol der Formel 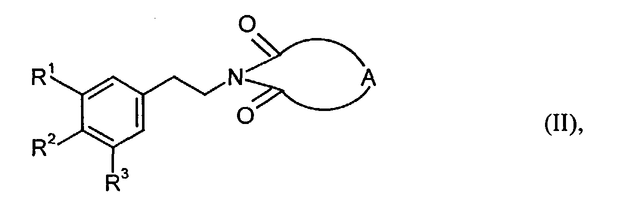 in der
- R¹, R² und R³: die bei Formel (I) angegebene Bedeutung und der Rest A die bei Formel (V) angegebene Bedeutung haben,
spaltet.

In den Formeln (I), (II), (III) und (IV) steht einer der Reste
R¹ und R² bevorzugt für einen Rest aus der Reihe Fluor, Trifluormethyl, Trifluormethoxy, Tetrafluorethoxy oder Pentafluorethoxy und der andere Rest bevorzugt für Wasserstoff oder es stehen
R¹ und R² bevorzugt gemeinsam für -O-CF₂-O-, -O-CF₂-CF₂- oder -O-CF₂-CF₂-O-.
R³ steht bevorzugt für Wasserstoff.
A in den Formeln (II), (III) und (V) steht bevorzugt für -CH₂-CH₂- oder o-Phenylen.

Besonders bevorzugt stehen in den Formeln (I), (II), (III) und (IV)
R¹ und R³ für Wasserstoff und R² für Fluor, Trifluormethyl oder Trifluormethoxy oder
R¹ für Fluor, Trifluormethyl oder Trifluormethoxy und R² und R³ für Wasserstoff oder
R¹ und R² gemeinsam für -O-CF₂-O-, -O-CF₂-CF₂- oder -O-CF₂-CF₂-O- und R³ für Wasserstoff.

In den Formeln (II), (III) und (V) steht A besonders bevorzugt für o-Phenylen.

Bei Tetrafluorethoxy-Resten handelt es sich vorzugsweise um 1,1,2,2-Tetrafluorethoxy-Reste.

Ganz besonders bevorzugt werden erfindungsgemäß die in Tabelle 1 angegebenen Fluor enthaltenden Phenethylamine hergestellt.

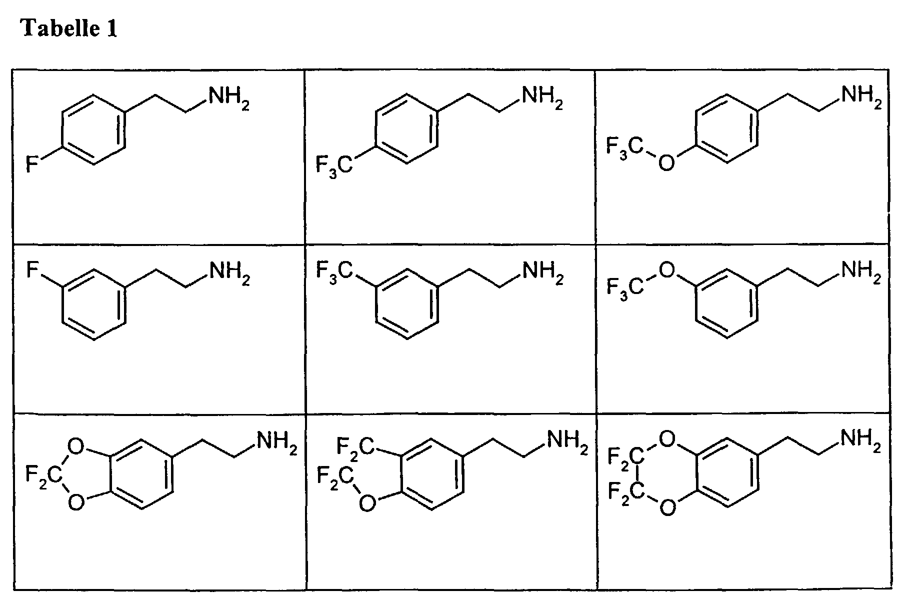

Die Umsetzung der Brombenzole der Formel (IV) mit N-Vinylimiden der Formel (V) zu den β-Iminovinylbenzolen der Formel (III) wird in Gegenwart eines Palladiumkatalysators durchgeführt, wobei vorzugsweise Verdünnungsmittel und Reaktionshilfsmittel zugegen sind.

Von den N-Vinylimiden der Formel (V) sind z.B. N-Vinylphthalimid und N-Vinylsuccinimid kommerziell erhältlich. N-Vinylmaleinimid ist beispielsweise gemäß Macromol. Rapid Commum. 15, 867-872 (1994) zugänglich. N-Vinylglutarimid und N-Vinyldiglykolimid sind ebenfalls bekannt und auf bekannte Weise zugänglich. N-Vinyl-3,3-dimethylglutarimid ist aus der kommerziell erhältlichen entsprechenden Dicarbonsäure analog dem Succinimid oder dem Glutarimid herstellbar.

Die Brombenzole der Formel (IV) sind entweder kommerziell erhältlich oder literaturbekannt und nach allgemeinen Verfahren der Chemie aromatischer Verbindungen erhältlich.

Als Palladiumkatalysator für die erste Reaktionsstufe geeignet sind beispielsweise Palladiumkomplexe mit Aryl- oder Alkylphosphinen als Liganden. Man kann sowohl die Komplexe als auch Palladium(II)-Salze und die freien Liganden einsetzen. Bevorzugt setzt man Palladium(II)-acetat und Tri-o-tolylphosphin ein.

Als Verdünnungsmittel für die erste Reaktionsstufe sind dipolare Lösungsmittel und diese enthaltende Mischungen, beispielsweise mit aliphatischen und/oder aromatischen Kohlenwasserstoffen und/oder Ethern geeignet. Beispiele für dipolare Lösungsmittel sind: Nitrile wie Acetonitril, Propionitril, n- und i-Butyronitril und Benzonitril, Amide wie Formamid, N-Methylformamid, N,N-Dimethylformamid, N,N-Dimethylacetamid und N-Methylpyrrolidon, Ester wie Methyl-, Ethyl- und Butylacetat, Sulfoxide wie Dimethylsulfoxid und Sulfone wie Sulfolan. Es können auch Mischungen von dipolaren Lösungsmitteln eingesetzt werden.

Als Reaktionshilfsmittel für die erste Reaktionsstufe kommen z.B. schwache anorganische oder organische Basen in Frage. Bevorzugt sind Erdalkalimetall- und Alkalimetallacetate, -carbonate und -hydrogencarbonate wie Natrium-, Kalium-, Calcium- und Ammoniumacetat, Natrium-, Kalium- und Ammoniumcarbonat, Natrium- und Kaliumhydrogencarbonat und tertiäre Amine wie Trimethylamin, Triethylamin und Tributylamin. Bevorzugt setzt man Natrium- oder Kaliumacetat ein.

Zur Durchführung der ersten Reaktionsstufe kann man z.B. das jeweilige Brombenzol der Formel (IV) und das jeweilige N-Vinylimid der Formel (V) in Mengen von 0,5 bis 2 Mol Brombenzol bezogen auf 1 Mol N-Vinylimid einsetzen. Vorzugsweise liegt diese Menge bei 0,9 bis 1,1 Mol. Besonders bevorzugt arbeitet man mit äquimolaren Mengen von Brombenzol der Formel (IV) und N-Vinylimid der Formel (V). Bezogen auf das Brombenzol kann man z.B. 0,01 bis 20 mmol, bevorzugt 0,1 bis 10 mmol Palladiumkatalysator und 1 bis 10 Äquivalente, bevorzugt 1 bis 3 Äquivalente Reaktionshilfsmittel einsetzen. Wenn man getrennt ein Palladium(II)-Salz und freie Phosphinliganden einsetzt kann das Molverhältnis von Palladium(II)-Salz zu Phosphinliganden z.B. 1:1,5 bis 1:10, bevorzugt 1:2 bis 1:4 betragen. Die Menge Verdünnungsmittel ist nicht kritisch. Bevorzugt setzt man pro Mol Brombenzol der Formel (IV) 100 bis 2000 ml ein.

Die Reaktionstemperatur in der ersten Verfahrensstufe kann in einem größeren Bereich variiert werden. Sie kann z.B. zwischen 50 und 180°C, bevorzugt zwischen 80 und 150°C liegen.

Die katalytische Hydrierung von β-Phthaliminovinylbenzolen der Formel (III) zu den β-Phthaliminoethylbenzolen der Formel (II) wird i.a. mit Wasserstoffgas und vorzugsweise in Gegenwart eines Verdünnungsmittels durchgeführt.

Als Katalysator zur Durchführung dieser Hydrierung sind beispielsweise Edelmetalle auf Trägermaterialien, insbesondere Palladium und Platin auf Kohle, Silikaten, Siliciumdioxid, Aluminiumoxid, Zeolithen, Bariumsulfat, Calciumcarbonat und Spinellen geeignet. Besonders bevorzugt ist Palladium auf Kohle. Bezogen auf den fertigen Katalysator kann er z.B. 0,1 bis 20 Gew.-% Edelmetall enthalten. Vorzugsweise liegt diese Menge bei 5 bis 15 Gew.-%.

Als Verdünnungsmittel für die zweite Reaktionsstufe kommen Wasser, organische Lösungsmittel und beliebige Mischungen davon in Betracht. Beispielhaft seien als organische Lösungsmittel genannt: aliphatische, alicyclische und aromatische Kohlenwasserstoffe wie Petrolether, Hexan, Heptan, Cyclohexan, Methylcyclohexan, Benzol, Toluol, Xylol und Decalin, Ether wie Diethyl-, Diisopropyl-, Methyl-t-butyl- und Methyl-t-amylether, Dioxan, Tetrahydrofuran, 1,2-Dimethoxyethan, 1,2-Diethoxyethan, Diethylglykoldimethylether und Anisol, Ester wie Methyl-, Ethyl- oder Butylacetat und Alkohole wie Methanol, Ethanol, n- und i-Propanol, n-, iso-, sek.- und tert.-Butanol, Ethandiol, Propan-1,2-diol, Ethoxyethanol, Methoxyethanol, Diethylenglykolmonomethylether und Diethylenglykolmonoethylether. Bevorzugtes Lösungsmittel ist Tetrahydrofuran.

Zur Durchführung der zweiten Reaktionsstufe kann man z.B. pro Mol β-Iminovinylbenzol der Formel (III) 0,01 bis 0,5 Mol, bevorzugt 0,05 bis 0,2 Mol Katalysator (gerechnet als Metall) und 1 bis 10 l Verdünnungsmittel einsetzen. Der Katalysator kann mehrmals hintereinander eingesetzt werden. Bei Ansätzen mit bereits benutztem Katalysator kann es zur Verlängerung der Reaktionszeit kommen. Dem kann man durch Zugabe von frischem Katalysator entgegensteuern, z.B. in einer Menge von 5 bis 25 Gew.-%, bezogen auf den gesamten eingesetzten Katalysator.

Die Reaktionstemperatur für die zweite Reaktionsstufe kann in einem größeren Bereich variiert werden. Sie kann z.B. zwischen 0 und 100°C, bevorzugt zwischen 20 und 70°C liegen. Der Wasserstoffdruck kann z.B. zwischen 1 und 100 bar, bevorzugt zwischen 5 und 50 bar betragen.

Die Spaltung der β-Phthaliminoethylbenzole der Formel (II) zu den Phenethylaminen der Formel (I) kann beispielsweise mit einer wäßrigen Base durchgeführt werden. Hierfür sind als Basen beispielsweise Hydrazinhydrat und wäßrige Alkalihydroxide geeignet. Bevorzugt wird Hydrazinhydrat, gegebenenfalls zusammen mit einem wäßrigen Alkalihydroxid eingesetzt. Wenn man Hydrazinhydrat und ein wäßriges Alkalihydroxid einsetzt ist es bevorzugt, die Spaltung mit Hydrazinhydrat zu beginnen und das wäßrige Alkalihydroxid erst später zuzusetzen.

Pro Mol β-Phthaliminoethylbenzol der Formel (II) kann man beispielsweise 1,2 bis 5 Äquivalente Hydrazin und gegebenenfalls 1 bis 30 Mol wäßriges Alkalihydroxid einsetzen. Vorzugsweise setzt man zunächst 1,5 bis 3 Äquivalente Hydrazinhydrat und später 5 bis 20 Mol wäßriges Alkalihydroxid pro Mol β-Phthaliminoethylbenzol der Formel (II) zu. Von den Alkalihydroxiden ist Kaliumhydroxid bevorzugt.

Die Temperatur bei der Imidspaltung kann in einem weiten Bereich variiert werden. Im allgemeinen arbeitet man bei erhöhter Temperatur. Nach oben ist die Temperatur durch den Siedepunkt der Reaktionsmischung begrenzt. Vorzugsweise arbeitet man bei 50 bis 100°C.

Der Wasseranteil der eingesetzten Base kann z.B. zwischen 30 und 70 (Gew.-%, vorzugsweise zwischen 40 und 60 Gew. -% liegen.

Die Umsetzungen der ersten und dritten Stufe des erfindungsgemäßen Verfahrens können bei Normaldruck oder unter erhöhtem Druck durchgeführt werden. Vorzugsweise wird bei Normaldruck gearbeitet.

Die Reaktionsdurchführung, Aufarbeitung und Isolierung der Reaktionsprodukte kann nach allgemein üblichen bekannten Methoden erfolgen. Die endprodukte werden vorzugsweise durch Kristallisation, Destillation oder durch Entfernung der flüchtigen Bestandteile, gegebenenfalls im Vakuum, gereinigt.

Die vorliegende Erfindung betrifft weiterhin β-Iminovinylbenzole der Formel 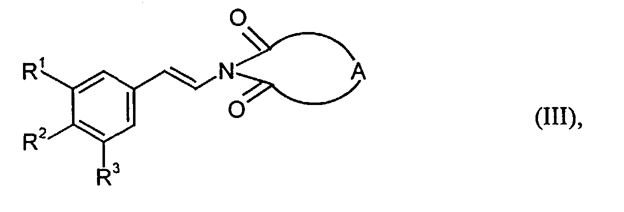 in der
R¹, R², R³ und A jeweils die breiteste oben angegebene Bedeutung haben,
mit Ausnahme der Verbindung N-[2-(3-Fluorphenyl)-ethenyl]-phthalimid. Die bevorzugten und besonders bevorzugten Bedeutungen dieser Reste sind ebenfalls entsprechend wie oben so angegeben.

Die vorliegende Erfindung betrifft schließlich auch β-Iminoethylbenzole der Formel 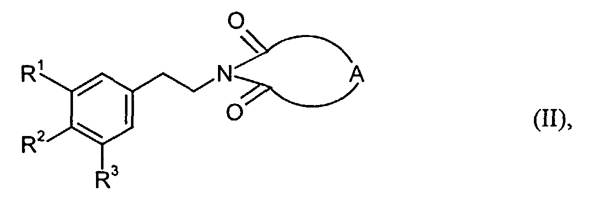 in der
R¹, R², R³ und A jeweils die breiteste oben angegebene Bedeutung haben. Die bevorzugten und besonders bevorzugten Bedeutungen dieser Reste sind ebenfalls entsprechend wie oben so angegeben.

Die neuen β-Iminovinyl- und β-Iminoethylbenzole können wie oben beschrieben hergestellt und als Zwischenprodukte, die nicht immer zwischenisoliert werden müssen, zur Herstellung von Fluor enthaltenden Phenethylaminen der Formel (I) verwendet werden. Die Zwischenisolierung kann insbesondere auf der Stufe der β-Iminoethylbenzole der Formel (II) unterbleiben.

Mit dem erfindungsgemäßen Verfahren gelingt die Herstellung von Fluor enthaltenden Phenethylaminen in einem dreistufigen Verfahren, bei dem die einzelnen Stufen mit Ausbeuten von 80 % und mehr realisierbar sind. Mit dem erfindungsgemäßen Verfahren wird erstmals ein auch in technischem Maßstab auf einfache Weise durchführbares Verfahren zur Herstellung von Fluor enthaltenden Phenethylaminen zur Verfügung gestellt. Besonderer sicherheitstechnischer Aufwand ist nicht erforderlich.

Das Auffinden der erfindungsgemäßen β-Iminovinyl- und β-Iminoethylbenzole machte die Konzeption des erfindungsgemäßen Verfahren im hier dargelegten Umfang erst möglich.

Die Existenz anderer Fluor enthaltender β-Iminovinylbenzole wird durch J. Org. Chem. 58, 3299 (1993) nicht nahegelegt, weil dort nur ein technisch und wirtschaftlich uninteressantes Verfahren zur Herstellung eines einzelnen β-Iminovinylbenzols beschrieben wird.

### Beispiele

### Beispiel 1 N-[2-(4-Trifluormethoxyphenyl)ethenyl]phthalimid

275 ml trockenes Dimethylformamid wurden 1 Stunde lang mit Stickstoff gespült. Dazu wurden nacheinander 100 g 1-Brom-4-trifluormethoxybenzol, 71,8 g N-Vinylphthalimid, 94,4 mg Palladium(II)acetat, 508 mg Tri(o-tolyl)phosphin und 67,8 g Natriumacetat gegeben und 24 Stunden bei 130°C gerührt. Nach dem Abkühlen wurde filtriert und das Filtrat eingeengt. Der Filtrationsrückstand wurde mit dem eingeengten Filtrat vereinigt, daß Gemisch in 300 ml Wasser suspendiert und erneut filtriert. Der jetzt vorliegende Filtrationsrückstand wurde mit weiteren 500 ml Wasser gewaschen und abgepreßt. Das Rohprodukt wurde in 200 ml n-Hexan suspendiert und 1 Stunde zum Sieden erhitzt. Es wurde heiß filtriert und der Filterrückstand getrocknet. Es wurden 126,9 g Produkt erhalten. Das Produkt war laut GC, angegeben in Flächenprozent, 92 %ig. Das entspricht einer Ausbeute von 84 % der Theorie.

### Beispiel 2 N-[2-(4-Trifluormethoxyphenyl)-ethyl]phthalimid

In einem Rührautoklaven wurden 200 g des gemäß Beispiel 1 erhaltenen Produkts gelöst in 500 ml Tetrahydrofuran vorgelegt, 16 g Palladium-auf-Kohle (10 gew.-%ig) zugesetzt und bei 50°C und einem Wasserstoffdruck von 10 bar bis zum vollständigen Umsatz (ca. 30 Stunden) hydriert. Danach wurde vom Katalysator abfiltriert und das Lösungsmittel entfernt. Es wurden 190 g Produkt erhalten. Das Produkt war laut GC, angegeben in Flächenprozent, 90 %ig. Die Ausbeute betrug 85% der Theorie.

Dieses Beispiel wurde zwei Mal wiederholt, wobei jeweils 85 Gew.-% bereits benutzter Katalysator und 15 Gew.-% frischer Katalysator zum Einsatz kamen. Die Ausbeute an Produkt änderte sich dabei praktisch nicht.

### Beispiel 3 2-(4-Trifluormethoxyphenyl)ethylamin

475 g des gemäß Beispiel 2 erhaltenen Produkts wurden in 2500 ml Toluol vorgelegt und auf 75°C erwärmt. Anschließend wurden 89 g Hydrazinmonohydrat zugetropft. Die Mischung wurde 8 Stunden unter Rückfluß gerührt. Danach wurden zu der Suspension bei 75°C 1000 g 50 gew.-%ige wäßrige Kalilauge getropft und weitere 4 Stunden unter Rückfluß gerührt. Nach dem Abkühlen wurde die wäßrige (schwerste) und eine organische, ölige (mittlere) Phase abgetrennt. Das Toluol als leichteste organische Phase wurde langsam abdestilliert und der Rückstand unter vermindertem Druck über eine kurze Vigreux-Kolonne destilliert. Es wurden bei 84 bis 86°C/11 mbar übergehend 216 g Produkt erhalten. Laut GC, angegeben in Flächenprozent, war es 99 %ig. Das entspricht einer Ausbeute von 80 % der Theorie.

### Beispiel 4 Herstellung des Vorprodukts N-Vinylphthalimid (nicht erfindungsgemäß)

Zu einer Lösung aus 1016 g N-(2-Bromethyl)phthalimid in 2,8 l N,N-Dimethylacetamid wurden über einen Zeitraum von 2 Stunden 672 g 1,8-Diazabicyclo-[5.4.0]-7-undecen getropft. Die Temperatur stieg dabei auf 60°C. Nach beendeter Zugabe wurde 12 Stunden nachgerührt. Der Ansatz wurde auf 91 Wasser gegossen, filtriert und der Filtrationsrückstand bei 50°C getrocknet. Es wurden 621 g Produkt (= 90 % der Theorie) mit einem Schmelzpunkt von 79 bis 80°C erhalten.

### Beispiel 5 N-{2-[3,4-Bis(trifluormethoxy)phenyl]ethenyl}phthalimid

7,3 l trockenes Dimethylformamid wurden 1 Stunde lang mit Stickstoff gespült. Dazu wurden nacheinander 2,71 kg 1-Brom-3,4-bis(trifluormethoxy)benzol, 1,95 kg N-Vinylphthalimid, 2,52 g Palladium(II)acetat, 13,7 g Tri-(o-tolyl)phosphin und 1,84 kg Natriumacetat gegeben und 30 Stunden bei 130°C gerührt. Nach dem Abkühlen wurde filtriert und das Filtrat eingeengt. Der Filtrationsrückstand wurde mit dem eingeengten Filtrat vereinigt, das Gemisch in 27 l Wasser suspendiert und erneut filtriert. Der jetzt vorliegende Filtrationsrückstand wurde mit 5 l n-Hexan gewaschen und abgepreßt. Es wurden 2,9 kg Produkt mit einem Schmelzpunkt von 159 bis 160°C erhalten. Das Produkt war laut GC, angegeben in Flächenprozent 90,5 %ig. Das entspricht einer Ausbeute von 70 % der Theorie.

### Beispiel 6 N-{2-[3,4-Bis(trifluormethoxy)pheny]ethyl}phthalimid

In einem Rührautoklaven wurden 5,65 kg des aus zwei Ansätzen gemäß Beispiel 21 erhaltenen Produkts gelöst in 14 kg Tetrahydrofuran vorgelegt, 452 g Palladium-auf-Kohle (10 Gew.-%ig) zugesetzt und bei 50°C und einem Wasserstoffdruck von 5 bis 10 bar bis zum vollständigen Umsatz (ca. 8 Stunden) hydriert. Danach wurde vom Katalysator abfiltriert und das Lösungsmittel entfernt. Es wurden 5 kg Produkt mit einem Schmelzpunkt von 91 bis 93°C erhalten. Das Produkt war laut GC, angegeben in Flächenprozent, 97,8 %ig. Die Ausbeute betrug 97 % der Theorie.

Dieses Beispiel wurde zwei Mal wiederholt, wobei jeweils 85 Gew.-% bereits benutzter Katalysator und 15 Gew.-% frischer Katalysator zum Einsatz kamen. Die Ausbeute an Produkt änderte sich dabei praktisch nicht.

### Beispiel 7 2-[3,4-Bis(trifluormethoxy)phenyl]ethylamin

500 g des gemäß Beispiel 6 erhaltenen Produkts wurden in 950 ml Toluol vorgelegt und auf 75°C erwärmt. Anschließend wurden 171,3 g Hydrazinmonohydrat zugetropft. Die Mischung wurde 8 Stunden bei 70 bis 80°C gerührt. Danach wurden 470 g 30 Gew.-%ige wäßrige Kalilauge zugetropft und weitere 60 min gerührt. Nach dem Abkühlen wurde die wäßrige (schwerste) und eine organische, ölige (mittlere) Phase abgetrennt. Das Toluol als leichteste organische Phase wurde langsam abdestilliert und der Rückstand unter vermindertem Druck über eine kurze Vigreux-Kolonne destilliert. Es wurden bei 85 bis 86°C/11 mbar übergehend 231,1 g Produkt erhalten. Laut GC, angegeben in Flächenprozent, war es 99,6 %ig. Das entspricht einer Ausbeute von 89 % der Theorie.

### Beispiel 8 Herstellung des Vorprodukts N-Vinylphthalimid (nicht erfindungsgemäß)

Zu einer Lösung aus 3,5 kg N-(2-Bromethyl)phthalimid in 9,6 l N,N-Dimethylacetamid wurden über einen Zeitraum von 2 Stunden 2,3 g 1,8-Diazabicyclo-[5.4.0]-7-undecen getropft. Die Temperatur stieg dabei auf 50°C. Nach beendeter Zugabe wurde 17 Stunden nachgerührt. Der Ansatz wurde auf 29 l Wasser gegossen, filtriert und der Filtrationsrückstand bei 30 bis 40°C getrocknet. Es wurden 1,95 kg Produkt (= 82 % der Theorie) mit einem Schmelzpunkt von 79 bis 80°C erhalten.

### Beispiel 9 N-[2-(3-Trifluormethylphenyl)ethylenyl]phthalimid

148 ml trockenes Dimethylformamid wurden 1 Stunde lang mit Stickstoff gespült. Dazu wurden nacheinander 50 g 1-Brom-3-trifluormethylbenzol, 38,4 g N-Vinylphthalimid, 503 mg Palladium(II)acetat und 2,72 g Tri(o-tolyl)phosphin gegeben. Bei 110°C wurden innerhalb 7 h 29,1 g Triethylamin hinzugetropft. Nach beendeter Zugabe wurde die Mischung 20 Stunden bei 110°C gerührt. Nach dem Abkühlen wurde die Reaktionsmischung eingeengt und der Rückstand mit 300 ml Wasser und 100 ml Methanol gerührt. Das Gemisch wurde filtriert und der Filtrationsrückstand mit 500 ml Wasser gewaschen und abgepreßt. Es wurden 55,3 g Produkt mit einem Schmelzpunkt von 139 bis 141°C erhalten. Das Produkt war laut GC, angegeben in Flächenprozent, 75,5 %ig. Das entspricht einer Ausbeute von 59 % der Theorie.

### Beispiel 10 N-[2-(3-Trifluormethylphenyl)ethyl]phthalimid

In einem Rührautoklaven wurden 50 g des gemäß Beispiel 9 erhaltenen Produkts gelöst in 150 ml Tetrahydrofuran vorgelegt, 2 g Palladium-auf-Kohle (10 Gew.-%ig) zugesetzt und bei 100°C und einem Wasserstoffdruck von 50 bar bis zum vollständigen Umsatz (ca. 21 Stunden) hydriert. Danach wurde vom Katalysator abfiltriert und das Lösungsmittel entfernt. Es wurden 44 g Produkt mit einem Schmelzpunkt von 69 bis 71°C erhalten. Das Produkt war laut GC, angegeben in Flächenprozent, 76,6 %ig. Die Ausbeute betrug 84 % der Theorie.

### Beispiel 11 2-(3-Trifluormethylphenyl)ethylamin

39,5 g des gemäß Beispiel 10 erhaltenen Produkts wurden in 80 ml Toluol vorgelegt und auf 75°C erwärmt. Anschließend wurden 16,2g Hydrazinmonohydrat zugetropft. Die Mischung wurde 3 Stunden bei 75°C gerührt. Danach wurden 37,8 g 25 Gew.-%ige wäßrige Kalilauge zugetropft und weitere 30 min gerührt. Nach dem Abkühlen wurde die wäßrige (schwerste) und eine organische, ölige (mittlere) Phase abgetrennt. Das Toluol als leichteste organische Phase wurde langsam abdestilliert und der Rückstand unter vermindertem Druck über eine kurze Vigreux-Kolonne destilliert. Es wurden bei 78 bis 80°C/15 mbar übergehend 10,5 g Produkt erhalten. Laut GC, angegeben in Flächenprozent, war es 97,3 %ig. Das entspricht einer Ausbeute von 60 % der Theorie.

### Beispiel 12 N-[2-(4-Trifluormethylphenyl)ethenyl]phthalimid

138 ml trockenes Dimethylformamid wurden 20 Minuten lang mit Stickstoff gespült Dazu wurden nacheinander 50 g 1-Brom-4-trifluormethylbenzol, 38,4 g N-Vinylphthalimid, 54,2 mg Palladium(II)acetat, 67,6 mg Tri(o-tolyl)phosphin und 36,4 g Natriumacetat gegeben und 12 Stunden bei 130°C gerührt. Nach dem Abkühlen wurde filtriert und das Filtrat eingeengt. Der Filtrationsrückstand wurde mit dem eingeengten Filtrat vereinigt, das Gemisch in 150 ml Wasser suspendiert und erneut filtriert. Der jetzt vorliegende Filtrationsrückstand wurde nacheinander mit 250 ml Wasser und 100 ml n-Hexan gewaschen und abgepreßt. Es wurden 36,5 g Produkt mit einem Schmelzpunkt von 201 bis 205°C erhalten. Das Produkt was laut GC, angegeben in Flächenprozent, 86,2 %ig. Das entspricht einer Ausbeute von 44 % der Theorie.

### Beispiel 13 N-[2-(4-Trifluormethylphenyl)ethyl]phthalimid

In einem Rührautoklaven wurden 31 g des gemäß Beispiel 12 erhaltenen Produkts gelöst in 300 ml Tetrahydrofuran vorgelegt, 2 g Palladium-auf-Kohle (10 Gew.-%ig) zugesetzt und bei 80°C und einem Wasserstoffdruck von 60 bar bis zum vollständigen Umsatz (ca. 8 Stunden) hydriert. Danach wurde vom Katalysator abfiltriert und das Lösungsmittel entfernt. Es wurden 32,8 g Produkt mit einem Schmelzpunkt von 134 bis 135°C erhalten. Das Produkt war laut GC, angegeben in Flächenprozent, 83,2 %ig. Die Ausbeute betrug 87 % der Theorie.

### Beispiel 14 2-(4-Trifluormethylphenyl)ethylamin

31,3 g des gemäß Beispiel 13 erhaltenen Produkts wurden in 80 ml Toluol vorgelegt und auf 75°C erwärmt. Anschließend wurden 13,1 g Hydrazinmonohydrat zugetropft. Die Mischung wurde 3 Stunden bei 75°C gerührt. Danach wurden 30,7 g 25 Gew.-%ige wäßige Kalilauge zugetropft und weitere 30 min gerührt. Nach dem Abkühlen wurde die wäßrige (schwerste) und eine organische, ölige (mittlere) Phase abgetrennt. Das Toluol als leichteste organische Phase wurde langsam abdestilliert und der Rückstand unter vermindertem Druck über eine kurze Vigreux-Kolonne destilliert. Es wurden bei 81 bis 82°C/12 mbar übergehend 9 g Produkt erhalten. Laut GC, angegeben in Flächenprozent, war es 97,6 %ig. Das entspricht einer Ausbeute von 57 % der Theorie.

### Beispiel 15 N-[2-(4-Fluorphenyl)ethenyl]phthalimid

190 ml trockenes Dimethylformamid wurden 20 Minuten lang mit Stickstoff gespült. Dazu wurden nacheinander 44,3g 1-Brom-4-fluorbenzol, 43,9 g N-Vinylphthalimid, 568 mg Palladium(II)acetat, und 3,1 g Tri(o-tolyl)phosphin. Bei 130°C wurden 33,2 g Triethylamin hinzugetropft. Nach beendeter Zugabe wurde 12 Stunden bei 130°C gerührt. Nach dem Abkühlen wurde die Reaktionsmischung eingeengt und der Rückstand in 200 ml Wasser suspendiert und filtriert. Der jetzt vorliegende Filtrationsrückstand wurde nacheinander mit 200 ml Wasser und 100 ml n-Hexan gewaschen und abgepreßt. Es wurden 41 g Produkt mit einem Schmelzpunkt von 151 bis 152°C erhalten. Das Produkt war laut GC, angegeben in Flächenprozent, 96,4 %ig. Das entspricht einer Ausbeute von 58 % der Theorie.

### Beispiel 16 N-[2-(4-Fluorphenyl)ethyl]phthalimid

In einem Rührautoklaven wurden 37 g des gemäß Beispiel 15 erhaltenen Produkts gelöst in 380 ml Tetrahydrofuran vorgelegt, 1,5 g Palladium-auf-Kohle (10 Gew. -%ig) zugesetzt und bei 60°C und einem Wasserstoffdruck von 50 bar bis zum vollständigen Umsatz (ca. 21 Stunden) hydriert. Danach wurde vom Katalysator abfiltriert und das Lösungsmittel entfernt. Es wurden 36,1 g des Produkts mit einem Schmelzpunkt von 106 bis 107°C erhalten. Das Produkt war laut GC, angegeben in Flächenprozent, 92,1 %ig. Die Ausbeute betrug 95 % der Theorie.

### Beispiel 17 2-(4-Fluorphenyl)ethylamin

34,6 g des gemäß Beispiel 16 erhaltenen Produkts wurden in 80 ml Toluol vorgelegt und auf 75°C erwärmt. Anschließend wurden 17,6 g Hydrazinmonohydrat zugetropft. Die Mischung wurde 3 Stunden bei 75°C gerührt. Danach wurden 41,6 g 25 Gew.-%ige wäßige Kalilauge zugetropft und weitere 30 min gerührt. Nach dem Abkühlen wurde die wäßrige (schwerste) und eine organische, ölige (mittlere) Phase abgetrennt. Das Toluol als leichteste organische Phase wurde langsam abdestilliert und der Rückstand unter vermindertem Druck über eine kurze Vigreux-Kolonne destilliert. Es wurden bei 83 bis 85°C/16 mbar übergehend 12 g Produkt erhalten. Laut GC, angegeben in Flächenprozent, war es 96,3 %ig. Das entspricht einer Ausbeute von 70 % der Theorie.

### Beispiel 18 N-[2-(3-Fluorphenyl)ethenyl]phthalimid

190 ml trockenes Dimethylformamid wurden 20 Minuten lang mit Stickstoff gespült. Danach wurden nacheinander 44,3g 1-Brom-3-fluorbenzol, 43,9 g N-Vinylphthalimid, 568 mg Palladium(II)acetat, und 3,1 g Tri(o-tolyl)phosphin zugefügt. Bei 130°C wurden 33,2 g Triethylamin hinzugetropft. Nach beendeter Zugabe wurde 16 Stunden bei 130°C gerührt. Nach dem Abkühlen wurde die Reaktionsmischung eingeengt und der Rückstand in 200 ml Wasser suspendiert und filtriert. Der jetzt vorliegende Filtrationsrückstand wurde nacheinander mit 200 ml Wasser und 100 ml n-Hexan gewaschen und abgepreßt. Es wurden 47,2 g Produkt mit einem Schmelzpunkt von 175 bis 177°C erhalten. Das Produkt war laut GC, angegeben in Flächenprozent, 86,7 %ig. Das entspricht einer Ausbeute von 60 % der Theorie.

### Beispiel 19 N-[2-(3-Fluorphenyl)ethyl]phthalimid

In einem Rührautoklaven wurden 43,2 g des gemäß Beispiel 18 erhaltenen Produkts gelöst in 300 ml Tetrahydrofuran vorgelegt, 1 g Palladium-auf-Kohle (10 Gew.-%ig) zugesetzt und bei 100°C und einem Wasserstoffdruck von 50 bar bis zum vollständigen Umsatz (ca. 37 Stunden) hydriert. Danach wurde vom Katalysator abfiltriert und das Lösungsmittel entfernt. Es wurden 41 g Produkt mit einem Schmelzpunkt von 122 bis 125°C erhalten. Das Produkt war laut GC, angegeben in Flächenprozent, 86 %ig. Die Ausbeute betrug 81 % der Theorie.

### Beispiel 20 2-(3-Fluorphenyl)ethylamin

37,4 g des gemäß Beispiel 19 erhaltenen Produkts wurden in 80 ml Toluol vorgelegt und auf 75°C erwärmt. Anschließend wurden 18,9 g Hydrazinmonohydrat zugetropft. Die Mischung wurde 3 Stunden bei 75°C gerührt. Danach wurden 44,9 g 25 Gew.-%ige wäßige Kalilauge zugetropft und weitere 30 min gerührt. Nach dem Abkühlen wurde die wäßrige (schwerste) und eine organische, ölige (mittlere) Phase abgetrennt. Das Toluol als leichteste organische Phase wurde langsam abdestilliert und der Rückstand unter vermindertem Druck über eine kurze Vigreux-Kolonne destilliert. Es wurden bei 79 bis 81 °C/15 mbar übergehend 10,6 g Produkt erhalten. Laut GC, angegeben in Flächenprozent, war es 94,7 %ig. Das entspricht einer Ausbeute von 52 % der Theorie.

### Beispiel 21 N-{2-[(3,4-Bis(trifluormethoxy)phenyl]ethenyl}phthalimid

160 ml trockenes Dimethylformamid wurden 20 Minuten lang mit Stickstoff gespült. Dazu wurden nacheinander 90 g 1-Brom-3,4-bis(trifluormethoxy)benzol, 48 g N-Vinylphthalimid, 33,8 mg Palladium(II)acetat, 84,2 mg Tri(o-tolyl)phosphin und 45 g Natriumacetat gegeben und 24 Stunden bei 130°C gerührt. Nach dem Abkühlen wurde filtriert und das Filtrat eingeengt. Der Filtrationsrückstand wurde mit dem eingeengten Filtrat vereinigt, das Gemisch in 1 l Wasser suspendiert und erneut filtriert. Der jetzt vorliegende Filtrationsrückstand wurde mit 200 ml n-Hexan gewaschen und abgepreßt. Es wurden 92 g Produkt mit einem Schmelzpunkt von 162 bis 164°C erhalten. Das Produkt war laut GC, angegeben in Flächenprozent, 93,5 %ig. Das entspricht einer Ausbeute von 74 % der Theorie.

### Beispiel 22 N-{2-[3,4-Bis(trifluormethoxy)phenyl]ethyl}phthalimid

In einem Rührautoklaven wurden 90 g des gemäß Beispiel 21 erhaltenen Produkts gelöst in 280 ml Tetrahydrofuran vorgelegt, 2 g Palladium-auf-Kohle (10 Gew.-%ig) zugesetzt und bei 50°C und einem Wasserstoffdruck von 10 bar bis zum vollständigen Umsatz (ca. 24 Stunden) hydriert. Danach wurde vom Katalysator abfiltriert und das Lösungsmittel entfernt. Es wurden 84 g Produkt mit einem Schmelzpunkt von 60 bis 61°C erhalten. Das Produkt war laut GC, angegeben in Flächenprozent, 90 %ig. Die Ausbeute betrug 84 % der Theorie.

### Beispiel 23 2-[3,4-Bis(trifluormethoxy)phenyl]ethylamin

66,7 g des gemäß Beispiel 22 erhaltenen Produkts wurden in 125 ml Toluol vorgelegt und auf 75°C erwärmt. Anschließend wurden 21,5 g Hydrazinmonohydrat zugetropft. Die Mischung wurde 8 Stunden bei 75°C gerührt. Danach wurden 53,9 g 25 Gew.-%ige wäßige Kalilauge zugetropft und weitere 30 min gerührt. Nach dem Abkühlen wurde die wäßrige (schwerste) und eine organische, ölige (mittlere) Phase abgetrennt. Das Toluol als leichteste organische Phase wurde langsam abdestilliert und der Rückstand unter vermindertem Druck über eine kurze Vigreux-Kolonne destilliert. Es wurden bei 97 bis 98°C/20 mbar übergehend 35,8 g Produkt erhalten. Laut GC, angegeben in Flächenprozent, war es 99,3 %ig. Das entspricht einer Ausbeute von 85 % der Theorie.

### Beispiel 24 N-[2-(2,2-Difluorbenzol[1,3]dioxol-5-yl)ethenyl}phthalimid

150 ml trockenes Dimethylformamid wurden 20 Minuten lang mit Stickstoff gespült. Dazu wurden nacheinander 32,6 g 5-Bromo-2,2-difluorbenzo-1,3-dioxol, 24,6 g N-Vinylphthalimid, 315 mg Palladium(II)acetat, 1,72 mg Tri(o-tolyl)phosphin und 23 g Natriumacetat gegeben und 24 Stunden bei 130°C gerührt. Nach dem Abkühlen wurde filtriert und das Filtrat eingeengt. Der Filtrationsrückstand wurde mit dem eingeengten Filtrat vereinigt, das Gemisch in 500 ml Wasser suspendiert und erneut filtriert. Der jetzt vorliegende Filtrationsrückstand wurde mit 100 ml n-Hexan gewaschen und abgepreßt. Es wurden 25,8 g Produkt mit einem Schmelzpunkt von 162 bis 164°C erhalten. Das Produkt war laut GC, angegeben in Flächenprozent, 94 %ig. Das entspricht einer Ausbeute von 54 % der Theorie.

### Beispiel 25 N-[2-(2,2-Difluorbenzol[1,3 ]dioxol-5-yl)ethyl]phthalimid

In einem Rührautoklaven wurden 24,2 g des gemäß Beispiel 24 erhaltenen Produkts gelöst in 60 ml Tetrahydrofuran vorgelegt, 2 g Palladium-auf-Kohle (10 Gew.-%ig) zugesetzt und bei 50°C und einem Wasserstoffdruck von 10 bar bis zum vollständigen Umsatz (ca. 6 Stunden) hydriert. Danach wurde vom Katalysator abfiltriert und das Lösungsmittel entfernt. Es wurden 22,5 g Produkt erhalten. Das Produkt war laut GC, angegeben in Flächenprozent, 44 %ig. Die Ausbeute betrug 41 % der Theorie.

### Beispiel 26 2-(2,2-Difluorbenzol[1,3]dioxol-5-yl)ethylamin

21 g des gemäß Beispiel 25 erhaltenen Produkts wurden in 80 ml Toluol vorgelegt und auf 75°C erwärmt. Anschließend wurden 8,5 g Hydrazinmonohydrat zugetropft. Die Mischung wurde 3 Stunden bei 75°C gerührt. Danach wurden 20 g 25 Gew.-%ige wäßige Kalilauge zugetropft und weitere 30 min gerührt. Nach dem Abkühlen wurde die wäßrige (schwerste) und eine organische, ölige (mittlere) Phase abgetrennt. Das Toluol als leichteste organische Phase wurde langsam abdestilliert. Der Rückstand wurde in 50 ml n-Hexan suspendiert und filtriert. Anschließend wurde das Filtrat eingeengt. Es wurden 3,7 g Produkt erhalten. Laut GC, angegeben in Flächenprozent, war es 87 %ig. Das entspricht einer Ausbeute von 58 % der Theorie.

### Beispiel 27 N-[2-(2,2,3,3-Tetrafluorbenzol[1,4]dioxin-6-yl)ethenyl]phthalimid

150 ml trockenes Dimethylformamid wurden 20 Minuten lang mit Stickstoff gespült. Dazu wurden nacheinander 39,5 g 6-Bromo-2,2,3,3-tetrafluorbenzo-1,4-dioxin, 24,6 g N-Vinylphthalimid, 315 mg Palladium(II)acetat, 1,72 mg Tri(o-tolyl)phosphin und 23 g Natriumacetat gegeben und 20 Stunden bei 130°C gerührt. Nach dein Abkühlen wurde filtriert und das Filtrat eingeengt. Der Filtrationsrückstand wurde mit dem eingeengten Filtrat vereinigt, das Gemisch in 500 ml Wasser suspendiert und erneut filtriert. Der jetzt vorliegende Filtrationsrückstand wurde mit 100 ml n-Hexan gewaschen und abgepreßt. Es wurden 44,3 g Produkt erhalten. Das Produkt war laut GC, angegeben in Flächenprozent, 98,5 %ig. Das entspricht einer Ausbeute von 83 % der Theorie.

### Beispiel 28 N-[2-(2,2,3,3-Tetrafluorbenzol[1,4]dioxin-6-yl)ethyl]phthalimid

In einem Rührautoklaven wurden 40 g des gemäß Beispiel 27 erhaltenen Produkts gelöst in 100 ml Tetrahydrofuran vorgelegt, 2 g Palladium-auf-Kohle (10 Gew.-%ig) zugesetzt und bei 50°C und einem Wasserstoffdruck von 10 bar bis zum vollständigen Umsatz (ca. 22 Stunden) hydriert. Danach wurde vom Katalysator abfiltriert und das Lösungsmittel entfernt. Es wurden 38,7 g Produkt erhalten. Das Produkt war laut GC, angegeben in Flächenprozent, 57 %ig. Die Ausbeute betrug 55 % der Theorie.

### Beispiel 29 2-(2,2,3,3-Tetrafluorbenzo[1,4]dioxin-6-yl)ethylamin

35 g des gemäß Beispiel 28 erhaltenen Produkts wurden in 80 ml Toluol vorgelegt und auf 75°C erwärmt. Anschließend wurden 12,2 g Hydrazinmonohydrat zugetropft. Die Mischung wurde 3 Stunden bei 75°C gerührt. Danach wurden 29,1 g 25 Gew.-%ige wäßige Kalilauge getropft und weitere 30 min gerührt. Nach dem Abkühlen wurde die wäßrige (schwerste) und eine organische, ölige (mittlere) Phase abgetrennt. Das Toluol als leichteste organische Phase wurde langsam abdestilliert. Der Rückstand wurde in 50 ml n-Hexan suspendiert und filtriert. Anschließend wurde das Filtrat eingeengt. Es wurden 8,7 g Produkt erhalten. Laut GC, angegeben in Flächenprozent, war es 90 %ig. Das entspricht einer Ausbeute von 60 % der Theorie.

### Beispiel 30 N-[2-(4-Trifluormethoxyphenyl)ethenyl]succinimid

55 ml trockenes Dimethylformamid wurden 30 Minuten lang mit Stickstoff gespült. Dazu wurden nacheinander 18,6 g 1-Brom-4-(trifluormethoxy)benzol, 9,7 g N-Vinylsuccinimid, 19,3 mg Palladium(II)acetat, 24 mg Tri(o-tolyl)phosphin und 13 g Natriumacetat gegeben und 20 Stunden bei 130°C gerührt. Nach dem Abkühlen wurde filtriert und das Filtrat eingeengt und der Rückstand in 300 ml Wasser suspendiert und filtriert. Der Filtrationsrückstand wurde mit 300 ml n-Hexan gewaschen und getrocknet. Es wurden 15,7 g Produkt mit einem Schmelzpunkt von 141 bis 143°C erhalten. Das Produkt war laut GC, angegeben in Flächenprozent, 94,6 %ig. Das entspricht einer Ausbeute von 67 % der Theorie.

### Beispiel 31 N-[2-(4-Trifluormethoxyphenyl)ethyl]succinimid

In einem Rührautoklaven wurden 21,4 g des gemäß Beispiel 30 erhaltenen Produkts gelöst in 100 ml Tetrahydrofuran vorgelegt, 0,5 g Palladium-auf-Kohle (10 Gew.-%ig) zugesetzt und bei 50°C und einem Wasserstoffdruck von 5 bis 10 bar bis zum vollständigen Umsatz (ca. 4 Stunden) hydriert. Danach wurde vom Katalysator abfiltriert und das Lösungsmittel entfernt. Es wurden 16,7 g Produkt mit einem Schmelzpunkt von 91 bis 93°C erhalten. Das Produkt war laut GC, angegeben in Flächenprozent, 95,9 %ig. Die Ausbeute betrug 78 % der Theorie.

### Beispiel 32 2-(4-Trifluormethoxyphenyl)ethylamin

15,4 g des gemäß Beispiel 31 erhaltenen Produkts wurden in 40 ml Toluol vorgelegt und auf 75°C erwärmt. Anschließend wurden 7,2 g Hydrazinmonohydrat zugetropft. Die Mischung wurde 8 Stunden bei 75°C gerührt. Danach wurden 17,3 g 25 Gew.-%ige wäßige Kalilauge zugetropft und weitere 60 min gerührt. Nach dem Abkühlen wurde die wäßrige (schwerste) und eine organische, ölige (mittlere) Phase abgetrennt. Das Toluol als leichteste organische Phase wurde langsam abdestilliert und der Rückstand unter vermindertem Druck über eine kurze Vigreux-Kolonne destilliert. Es wurden bei 82 bis 84°C/12 mbar übergehend 6,4 g Produkt erhalten. Laut GC, angegeben in Flächenprozent, war es 99,1 %ig. Das entspricht einer Ausbeute von 61 % der Theorie.

## Patentansprüche

1. Verfahren zur Herstellung von Fluor enthaltenden Phenethylaminen der Formel 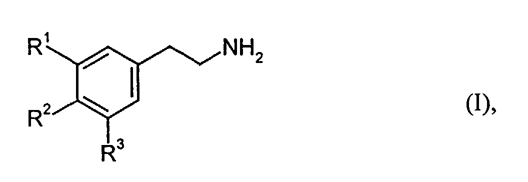 in der von den Resten
R¹ und R² einer für Fluor, Trifluormethyl, Trifluormethoxy, Tetrafluorethoxy oder Pentafluorethoxy und der andere für Wasserstoff, C₁-C₆-Alkyl, C₁-C₆-Alkoxy, Fluor, Trifluormethyl, Trifluormethoxy, Tetrafluorethoxy oder Pentafluorethoxy steht oder
R¹ und R² gemeinsam für -O-CF₂-O-, -O-CF₂-CF₂- oder -O-CF₂-CF₂-O-stehen und
R³ für Wasserstoff, C₁-C₆-Alkyl oder C₁-C₆-Alkoxy steht,
dadurch gekennzeichnet, daß man in einer ersten Stufe ein substituiertes Brombenzol der Formel 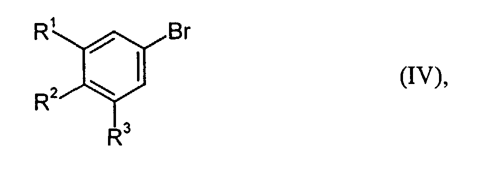 in der
R¹, R² und R³ die bei Formel (I) angegebene Bedeutung haben,
mit einem N-Vinylimid der Formel 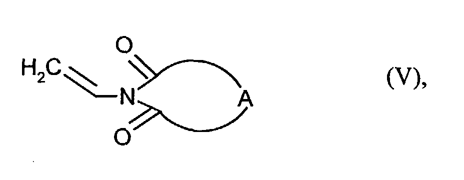 in der
A für -CH₂-CH₂-, -CH₂-CH₂-CH₂-, -CH₂-C(CH₃)₂-CH₂-, -CH₂-O-CH₂-, -CH=CH- oder o-Phenylen steht,
in Gegenwart eines Palladium-Katalysators umsetzt, in einer zweiten Stufe das erhaltene β-Iminovinylbenzol der Formel 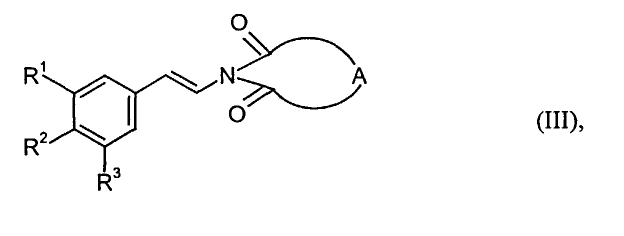 in der
die Reste R¹, R² und R³ die bei Formel (I) und der Rest A die bei Formel (V) angegebene Bedeutung haben,
katalytisch hydriert und in einer dritten Stufe das in der zweiten Stufe erhaltene substituierte β-Iminoethylbenzol der Formel 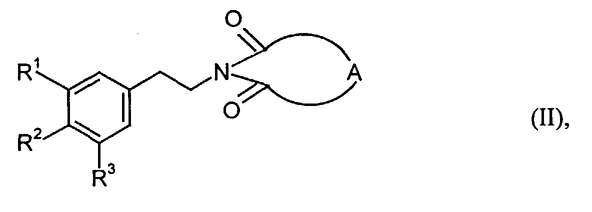 in der
R¹, R² und R³ die bei Formel (I) angegebene Bedeutung und
A die bei Formel (V) angegebene Bedeutung haben,
spaltet.

2. Verfahren nach Anspruch 1, dadurch gekennzeichnet, daß in den Formeln (I), (II), (III) und (IV) einer der Reste R¹ und R² für einen Rest aus der Reihe Fluor, Trifluormethyl, Trifluormethoxy, Tetrafluorethoxy oder Pentafluorethoxy und der andere Rest für Wasserstoff steht oder
R¹ und R² gemeinsam für -O-CF₂-O-, -O-CF₂-CF₂- oder -O-CF₂-CF₂-O-stehen,
R³ für Wasserstoff steht und
A in den Formeln (II), (III) und (V) für -CH₂-CH₂- oder o-Phenylen steht.

3. Verfahren nach Ansprüchen 1 und 2, dadurch gekennzeichnet, daß man in die erste Stufe als Palladiumkatalysator Palladiumkomplexe mit Aryl- oder Alkylphosphinen als Liganden, dipolare Lösungsmittel als Verdünnungsmittel und schwache anorganische oder organische Basen als Reaktionshilfsmittel einsetzt und die Reaktion bei Temperaturen zwischen 50 und 180°C durchführt.

4. Verfahren nach Ansprüchen 1 bis 3, dadurch gekennzeichnet, daß man die zweite Stufe mit Wasserstoff in Gegenwart eines Verdünnungsmittels und in Gegenwart von Edelmetallen auf Trägermaterialien als Katalysator durchführt.

5. Verfahren nach Ansprüchen 1 bis 4, dadurch gekennzeichnet, daß man die zweite Reaktionsstufe in Gegenwart von Wasser oder organischen Lösungsmitteln als Verdünnungsmittel durchführt, pro Mol β-Iminovinylbenzol der Formel (III) 0,01 bis 0,5 Mol Katalysator (gerechnet als Metall) einsetzt und eine Reaktionstemperatur zwischen 0 und 100°C anwendet.

6. Verfahren nach Ansprüchen 1 bis 5, dadurch gekennzeichnet, daß man die dritte Stufe mit wäßrigen Basen und bei Temperaturen von 50 bis 100°C durchführt.

7. Verfahren nach Ansprüchen 1 bis 6, dadurch gekennzeichnet, daß man in die dritte Stufe pro Mol Imid der Formel (II) zwischen 1, 2 und 5 Äquivalente Hydrazinhydrat und gegebenenfalls zwischen 1 und 30 Mol wäßriges Alkalihydroxid einsetzt.

8. β-Iminovinylbenzole der Formel 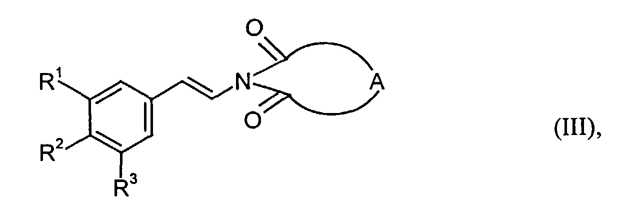 in der von den Resten
R¹ und R² einer für Fluor, Trifluormethyl, Trifluormethoxy, Tetrafluorethoxy oder Pentafluorethoxy und der andere für Wasserstoff, C₁-C₆-Alkyl, C₁-C₆-Alkoxy, Fluor, Trifluormethyl, Trifluormethoxy, Tetrafluorethoxy oder Pentafluorethoxy steht oder
R¹ und R² gemeinsam für -O-CF₂-O-, -O-CF₂-CF₂- oder -O-CF₂-CF₂-O-stehen,
R³ für Wasserstoff, C₁-C₆-Alkyl oder C₁-C₆-Alkoxy steht und
A für -CH₂-CH₂-, -CH₂-CH₂-CH₂-, -CH₂C(CH₃)₂-CH₂-, -CH₂-O-CH₂-, -CH=CH- oder o-Phenylen steht,
mit Ausnahme der Verbindung N-[2-(3-Fluorphenyl)-ethenyl]-phthalimid.

9. β-Iminoethylbenzole der Formel 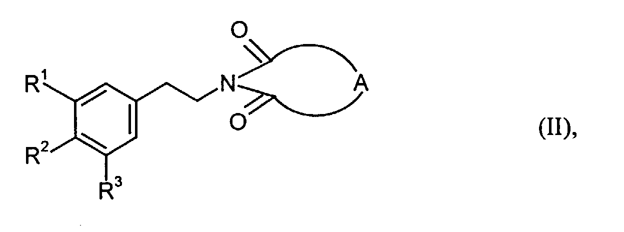 in der von den Resten
R¹ und R² einer für Fluor, Trifluormethyl, Trifluormethoxy, Tetrafluorethoxy oder Pentafluorethoxy und der andere für Wasserstoff, C₁-C₆-Alkyl, C₁-C₆-Alkoxy, Fluor, Trifluormethyl, Trifluormethoxy, Tetrafluorethoxy oder Pentafluorethoxy steht oder
R¹ und R² gemeinsam für -O-CF₂-O-, -O-CF₂-CF₂- oder -O-CF₂-CF₂-O-stehen und
R³ für Wasserstoff, C₁-C₆-Alkyl oder C₁-C₆-Alkoxy steht, und
A für -CH₂-CH₂-, -CH₂-CH₂-CH₂-, -CH₂-C(CH₃)₂-CH₂-, -CH₂-O-CH₂-, -CH=CH- oder o-Phenylen steht.
